# EUROPÄISCHE PATENTANMELDUNG

(11) **EP 4 257 683 A1**
(43) Veröffentlichungstag der Anmeldung: **11.10.2023**
(21) Anmeldenummer: 22167501.0
(22) Anmeldetag: 08.04.2022
(51) Int. Cl.: C12N 9/80

(54) **NEUE URETHANASEN FÜR DEN ENZYMATISCHEN ABBAU VON POLYURETHANEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft neue Urethanasen für den enzymatischen Abbau von Polyurethanen sowie ein enzymatisches Verfahren für den vollständigen Abbau von Polyurethanen in definierte Monomere.

## Beschreibung

Die vorliegende Erfindung betrifft neue Urethanasen für den enzymatischen Abbau von Polyurethanen sowie ein enzymatisches Verfahren für den vollständigen Abbau von Polyurethanen in definierte Monomere.

Polyurethane sind in vielen Bereichen des normalen Lebens etabliert. Sie finden sich zum Beispiel in Weichschäumen (Matratzen, Schwämme, Polstermöbel), Hartschäumen (Isolationsmaterialien, Baustoffe), Thermoplasten (Sportschuhe) oder Beschichtungen (Lacke, Farben, Klebstoffe). Aufgrund der stetig steigenden Nachfrage nach den Produkten werden zunehmend größere Mengen produziert. Parallel dazu wächst das Bedürfnis nach Methoden für möglichst nachhaltiges Recycling von nicht mehr benötigten Polyurethanprodukten, die eine Wiederverwertung von Bausteinen der Polymere erlaubt. Dafür müssen die Bindungen in den Polyurethanen gezielt gespalten werden, um definierte Abbauprodukte erhalten zu können und diese damit wiederverwertbar zu machen.

Neben den physiologischen Funktionen, welche Enzyme in lebenden Organismen erfüllen, können sie auf vielfältige Weise zur Katalyse chemischer Reaktionen außerhalb dieses Kontextes genutzt werden. Hierbei können im Vergleich zu konventionell chemischen Prozessen die Reaktionen bei milderen Bedingungen wie niedrigerer Temperatur, neutralem pH-Wert und ohne die Verwendung aggressiver Chemikalien durchgeführt werden. Dadurch kann Energie gespart, die Bildung von Nebenprodukten minimiert und die Umwelt geschont werden, was zur Reduktion der Betriebskosten beiträgt. Durch den Einsatz von Enzymen wird es teilweise erst ermöglicht, dass labile Edukte umgesetzt werden können (Jaeger, K.-E. & Reetz, M. T. (1998) Microbial lipases form versatile tools for biotechnology. Trends in biotechnology, 16, 396-403). Weiterhin sind Enzyme häufig chemo-, regio-, stereo- und enantioselektiv, was die Aufreinigung der Produkte deutlich erleichtert und damit die effiziente Synthese schwer zugänglicher Produkte ermöglichen kann (Hasan, F., Shah, A. A. & Hameed, A. (2006) Industrial applications of microbial lipases. Enzyme and Microbial Technology, 39, 235-251).

Heutzutage werden Polyurethane in den wenigsten Fällen recycelt. Die Entsorgung geschieht durch Deponierung oder alternativ durch eine vollständige Verbrennung, wobei zwar Energie gewonnen wird, aber eine effiziente Wiederverwendung der Polymerbausteine entfällt.

Es ist bekannt, dass Polyurethane in gewissem Maße von Bakterien und Pilzen abgebaut werden können. Dabei sind Polyesterpolyurethane deutlich anfälliger gegenüber mikrobiellem bzw. enzymatischem Abbau als Polyetherpolyurethane (Nakajima-Kambe, T., Shigeno-Akutsu, Y., Nomura, N., Onuma, F. & Nakahara, T. (1999) Microbial degradation of polyurethane, polyester polyurethanes and polyether polyurethanes. Applied microbiology and biotechnology, 51, 134-140).

Der Abbau von Polyesterpolyurethanen lässt sich leicht über die Hydrolyse der Esterbindungen realisieren. Der relativ einfache Abbau von Polyestern ist nicht weiter verwunderlich, da Esterbindungen in hydrophoben Substraten in der Natur auch beim Abbau von Lipiden gespalten werden müssen und auch Polyester ohne Urethanbindungen relativ leicht durch Esterasen und Lipasen abgebaut werden können (Marten, E., Müller, R.-J. & Deckwer, W.-D. (2003) Studies on the enzymatic hydrolysis of polyesters I. Low molecular mass model esters and aliphatic polyesters. Polymer degradation and stability, 80, 485-501; Marten, E., Müller, R.-J. & Deckwer, W.-D. (2005) Studies on the enzymatic hydrolysis of polyesters. II. Aliphatic-aromatic copolyesters. Polymer degradation and stability, 88, 371-381.). In verschiedenen Literaturstellen wurden zum Abbau von Polyurethan verwendete Enzyme als Esterasen charakterisiert (Allen, A. B., Hilliard, N. P. & Howard, G. T. (1999) Purification and characterization of a soluble polyurethane degrading enzyme from Comamonas acidovorans. International biodeterioration & biodegradation, 43, 37-41; Blake, R., Norton, W. & Howard, G. (1998) Adherence and growth of a Bacillus species on an insoluble polyester polyurethane. International biodeterioration & biodegradation, 42, 63-73; Crabbe, J. R., Campbell, J. R., Thompson, L., Walz, S. L. & Schultz, W. W. (1994) Biodegradation of a colloidal ester-based polyurethane by soil fungi. International biodeterioration & biodegradation, 33, 103-113; Darby, R. T. & Kaplan, A. M. (1968) Fungal susceptibility of polyurethanes. Applied microbiology, 16, 900-905; Howard, G. T., Norton, W. N. & Burks, T. (2012) Growth of Acinetobacter gerneri P7 on polyurethane and the purification and characterization of a polyurethanase enzyme. Biodegradation, 23, 561-573; Kaplan, A. M., Darby, R. T., Greenberger, M. & Rodgers, M. (1968) Microbial deterioration of polyurethane systems. Dev Ind Microbiol, 82, 362-371; Kay, M., Morton, L. & Prince, E. (1991) Bacterial degradation of polyester polyurethane. International biodeterioration, 27, 205-222; Vega, R. E., Main, T. & Howard, G. T. (1999) Cloning and expression in Escherichia coli of a polyurethane-degrading enzyme from Pseudomonas fluorescens. International biodeterioration & biodegradation, 43, 49-55). Dort gibt es keinen eindeutigen Nachweis auf die Spaltung der Urethanbindung, da die Enzymcharakterisierung in keinem Fall auf Grundlage der Spaltung eines Moleküls mit Urethangruppe durchgeführt wurde.

In vielen Publikationen und Patenten ist der Abbau von Poly(ester)urethanen mit Pilzen oder Bakterien beschrieben. Jedoch zielt der Abbau meist nur auf die relativ leicht zu spaltenden Esterbindungen und wird meist nur durch eine makroskopische Beobachtung des Polymerabbaus belegt. Es gibt hier keinen gesteuerten Abbau von Ester- und Urethanbindungen, wie in der vorliegenden Erfindung und es ergeben sich häufig lange Abbauzeiten. Diese Veröffentlichungen zeigen, dass Urethanasen weit verbreitete Enzyme sind, jedoch belegen sie nicht deren spezifischen Fähigkeiten, Einsatzmöglichkeiten und Gruppenzugehörigkeit, wie in der vorliegenden Erfindung genutzt. (JP09192633, Tang, Y. W., Labow, R. S., Santerre, J. P. (2003) Enzyme induced biodegradation of polycarbonate-polyurethanes: dose dependence effect of cholesterol esterase. Biomaterials 24 (12), 2003-2011, Vega, R. E., Main, T. & Howard, G. T. (1999) Cloning and expression in Escherichia coli of a polyurethane-degrading enzyme from Pseudomonas fluorescens. International biodeterioration & biodegradation, 43, 49-55)

Zum enzymatischen Abbau von Poly(ester)urethanen ist ein Abbauverfahren bekannt, bei dem im ersten Schritt aus einer Kultivierung von *Comamonas acidovorans* eine Esterase gewonnen wird, indem als Kohlenstoffquelle nur Poly(ester)urethan zur Verfügung steht. In einem aufwendigen Aufarbeitungsschritt wird die Esterase abgetrennt und für den Abbau von Poly(ester)urethanen im Batch eingesetzt. Hierbei ergeben sich lange Abbauzeiten in einem mehrstufigen Verfahren und keine Belege für eine spezifische Spaltung der Urethanbindungen. (JP 09201192, JP 10271994)

In verschiedenen Patenten und Publikationen wird der Abbau von Poly(ester)urethanen mit Cutinasen, Esterasen und/oder Lipasen beschrieben. Jedoch zielt der Abbau auch hier nur auf die relativ einfache Spaltung der Esterbindungen, nicht aber spezifisch auf die Urethanbindungen. Außerdem wird keine gezielte Kombination von Ester- und Urethanbindungen spaltenden Enzymen zur gezielten Steuerung des Abbaus beschrieben. Es ist davon auszugehen, dass die beschriebenen Verfahren nur zu einer geringen oder keiner Spaltung der Urethanbindung führen. Damit können in der Synthese von Polyurethanen eingesetzte Diamine nicht effizient wiedergewonnen werden. (EP 0968300, US 6,180,381)

In WO 2013/134801 wird der Abbau von aromatischen Polyurethanen, basierend auf Polyetherpolyolen, unter Anwendung eines Enzymes der Klasse EC 3 beschrieben. Dabei sind keine spezifischen Enzymsequenzen angegeben, so dass die Spezifität des Verfahrens in dem Abbau bestimmter Urethanbindungen, sowie der kontrollierten Spaltung von Ester- und gesondert der Spaltung von Urethanbindungen, wie in der vorliegenden Erfindung gezeigt, in dem genannten Patent nicht belegt ist.

In WO 2006/019095 wird eine Urethanase und durch Protein Engineering gewonnene Varianten dieses Enzyms beschrieben. Das Enzym kann auf TDA oder MDA beruhende Urethanoligomere spalten. Weiterhin werden in WO 2019/243293 neu identifizierte Urethanasen beschrieben.

Der vorliegenden Erfindung lag somit die Aufgabe zugrunde, weitere Enzyme bereitzustellen, die für die enzymatische Spaltung von Urethanbindungen und vorzugsweise für die Verwendung beim vollständigen enzymatischen Abbau von Polyurethanen einsetzbar sind. Weiterhin sollte ein enzymatisches Verfahren bereitgestellt werden, das den Abbau von Polyurethanen in definierte Monomere ermöglicht.

Diese Aufgabe wird durch die in den Patentansprüchen und in der untenstehenden Beschreibung offenbarten Ausführungsformen gelöst.

In einer ersten Ausführungsform betrifft die vorliegende Erfindung ein Polypeptid mit einer Aminosäuresequenz wie in SEQ ID NO.: 1 definiert oder eine Variante davon, dadurch gekennzeichnet, dass das Polypeptid Urethanaseaktivität aufweist.

### Polypeptid

Der Begriff "Polypeptid" ist dem Fachmann gut bekannt. Er bezeichnet eine Kette von wenigstens 50, vorzugsweise wenigstens 70 Aminosäuren, die durch Peptidbindungen miteinander verknüpft sind. Ein Polypeptid kann sowohl natürlich vorkommende als auch synthetische Aminosäuren enthalten. Vorzugsweise enthält es die bekannten proteinogenen Aminosäuren.

Eine Variante wird vorzugsweise durch Hinzufügung, Deletion oder Austausch von bis zu 15 %, stärker bevorzugt bis zu 10 % und noch stärker bevorzugt bis zu 5 %, der im jeweiligen Polypeptid enthaltenen Aminosäuren erhalten. Basis für die Berechnung der Sequenzidentität ist bevorzugt die durch SEQ ID NO.: 1 definierte Aminosäuresequenz. Dem Fachmann ist bewusst, dass Fusionsproteine von Enzymen mit anderen Proteinen gebildet werden, ohne dass dies die Aktivität des Enzyms beeinträchtigt. Deswegen umfasst der Begriff "Variante" auch solche von dem durch SEQ ID NO.: 1 definierten Polypeptid abgeleiteten Aminosäuresequenzen, die am N-Terminus und/oder am C-Terminus mit weiteren Proteinen, z.B. dem Green Fluorescent Protein, fusioniert sind.

Besonders bevorzugte Varianten des Polypeptids werden durch Hinzufügung, Deletion oder Austausch von bis zu 20, bevorzugt bis zu 10 und noch stärker bevorzugt bis zu 5 Aminosäuren der offenbarten Sequenzen erhalten. Grundsätzlich können die vorgenannten Modifikationen an jeder beliebigen Stelle des Polypeptids kontinuierlich oder diskontinuierlich erfolgen. Bevorzugt erfolgen sie aber nur am N-Terminus und/oder am C-Terminus des Polypeptids. Jede erfindungsgemäße durch Hinzufügung, Austausch oder Deletion von Aminosäuren erhaltene Variante ist aber durch Urethanaseaktivität wie weiter unten in dieser Anmeldung definiert gekennzeichnet.

### Urethanaseaktivität

Der Begriff "Urethanaseaktivität" bezieht sich auf die Fähigkeit eines Polypeptids, die Spaltung einer Urethangruppe enzymatisch zu katalysieren. Pro Mol Urethangruppe entsteht hierbei ein Mol Amin, ein Mol Alkohol und ein Mol CO₂.

Bei der Urethangruppe kann es sich um eine aromatisch, eine cycloaliphatisch oder eine aliphatisch gebundene Urethangruppe handeln. Bei einer aromatisch gebundenen Urethangruppe ist das Stickstoffatom direkt an einen aromatischen Ring gebunden. Bei einer aliphatisch gebundenen Urethangruppe ist das Stickstoffatom an einen Alkylrest, der Bestandteil eines offenkettigen Alkylrests ist, gebunden. Vorzugsweise handelt es sich um einen unverzweigten Alkylrest mit wenigstens einem, stärker bevorzugt wenigstens zwei und am stärksten bevorzugt wenigstens drei Kohlenstoffatomen. Bei einer cycloaliphatisch gebundenen Urethangruppe ist das Stickstoffatom an ein Kohlenstoffatom gebunden, das Bestandteil eines aliphatischen Rings ist. Dieser Ring kann an einer oder mehreren Stellen ungesättigt sein, solange er durch das Vorliegen von Doppelbindungen keinen aromatischen Charakter erhält. In einer bevorzugten Ausführungsform der vorliegenden Erfindung ist das Polypeptid mit Urethanaseaktivität in der Lage, eine aromatisch gebundene Urethangruppe enzymatisch zu spalten.

Ob ein Polypeptid eine Urethanaseaktivität besitzt, kann durch die Spaltung geeigneter Modellsubstrate überprüft werden. Dies sind vorzugsweise die Substrate, deren Spaltung in den Ausführungsbeispiel gezeigt wurde.

Für die Fähigkeit zur Spaltung aromatisch gebundener Urethangruppen dienen besonders bevorzugt Ethyl-4-Nitrophenylcarbamat (ENPC) oder das Carbamat, das durch die Reaktion von 7-Amino-4-Methylcumarin mit Ethylchlorformiat erhältlich ist (EMACC) als Modellsubstrate. Die Spaltung von ENPC wird durch die Bestimmung des Konzentrationsanstiegs von 4-Nitroanilin nachgewiesen. Dies erfolgt vorzugsweise photometrisch bei einer Wellenlänge von 405 nm. Die Enzymaktivität wird vorzugsweise in einem Rektionspuffer mit 100 mM K₂HPO₄/KH₂PO₄, pH 7 mit 6,25 Vol.-% Ethanol in Anwesenheit von 0,2 mg/L ENPC als Substrat bestimmt. Die Inkubation des Enzyms im Reaktionspuffer mit ENPC erfolgt vorzugsweise bei Raumtemperatur und vorzugsweise für 24 Stunden.

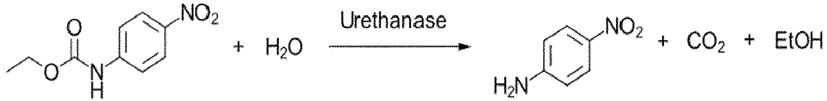

Die Spaltung von EMACC wird durch die Bestimmung des Konzentrationsanstiegs von 7-Amino-4-Methylcumarin (AMC) nachgewiesen. Dies erfolgt vorzugsweise photometrisch bei einer Wellenlänge von 365 nm. Die Enzymaktivität wird vorzugsweise in einem Rektionspuffer mit 50 mM Na₂HPO₄/NaH₂PO₄, pH 8,0 mit 0,2 Vol.-% DMSO in Anwesenheit von 100 µM EMACC als Substrat bestimmt. Die Inkubation des Enzyms im Reaktionspuffer mit ENPC erfolgt vorzugsweise bei 30 °C und vorzugsweise für bis zu 24 Stunden.

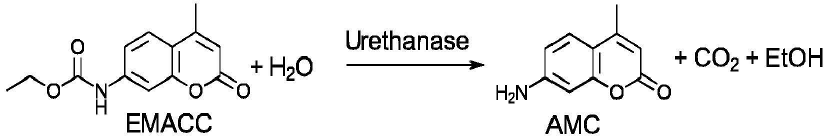

Für die Fähigkeit zur Spaltung aliphatisch gebundener Urethangruppen dient vorzugsweise Ethylphenethylcarbamat (EPEC) als Modellsubstrat. Die Spaltung wird durch die Bestimmung des Konzentrationsanstiegs von Phenethylamin nachgewiesen. Dies erfolgt vorzugsweise durch HPLC. Der verwendete Reaktionspuffer und die Reaktionsbedingungen entsprechen vorzugsweise den oben für ENPC beschriebenen Parametern.

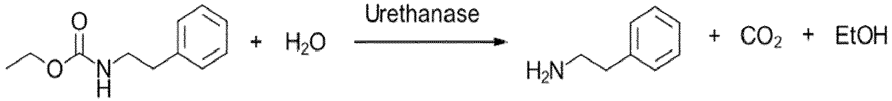

Die Urethanbindungen, die durch die erfindungsgemäßen Urethanasen gespalten werden können, basieren formal auf der Addition eines Polyisocyanats mit aliphatisch, cycloaliphatisch, araliphatisch oder aromatisch gebundenen Isocyanatgruppen und einem Alkohol.

Bei einem Isocyanat mit aliphatisch gebundenen Isocyanatgruppen sind alle Isocyanatgruppen an ein Kohlenstoffatom gebunden, das Teil einer offenen Kohlenstoffkette ist. Diese kann an einer oder mehreren Stellen ungesättigt sein. Die aliphatisch gebundene Isocyanatgruppe oder - im Fall von Polyisocyanaten - die aliphatisch gebundenen Isocyanatgruppen sind vorzugsweise an den terminalen Kohlenstoffatomen der Kohlenstoffkette gebunden.

Bevorzugte Polyisocyanate mit aliphatisch gebundenen Isocyanatgruppen sind 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan und 1,10-Diisocyanatodecan.

Bei einem Isocyanat mit cycloaliphatisch gebundenen Isocyanatgruppen sind alle Isocyanatgruppen an Kohlenstoffatome gebunden, die Teil eines geschlossenen Rings aus Kohlenstoffatomen sind. Dieser Ring kann an einer oder mehreren Stellen ungesättigt sein, solange er durch das Vorliegen von Doppelbindungen keinen aromatischen Charakter erhält.

Bevorzugte Polyisocyanate mit cycloaliphatisch gebundenen Isocyanatgruppen sind 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan Isophorondiisocyanat; (IPDI), 1-Isocyanato-1-methyl-4(3)-isocyanatomethylcyclohexan, 2,4'- und 4,4'-Diisocyanatodicyclohexylmethan (H12MDI), 1,3-und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyl-dicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan und 1,3-Dimethyl-5,7-diisocyanatoadamantan.

Bei einem Isocyanat mit araliphatisch gebundenen Isocyanatgruppen sind alle Isocyanatgruppen an Methylenreste gebunden, der ihrerseits an einen aromatischen Ring gebunden sind.

Bevorzugte Polyisocyanate mit araliphatisch gebundenen Isocyanatgruppen sind 1,3- und 1,4-Bis-(iso-cyanatomethyl)benzol (Xylylendiisocyanat); XDI), 1,3- und 1,4-Bis(1-isocyanato-1-methy¬ethy)-benzol (TMXDI) und Bis(4-(1-isocyanato-1-methylethyl)phenyl)-carbonat.

Bei einem Isocyanat mit aromatisch gebundener Isocyanatgruppe sind alle Isocyanatgruppen direkt an Kohlenstoffatome gebunden, die Teil eines aromatischen Ringes sind.

Bevorzugte Isocyanate mit aromatisch gebundenen Isocyanatgruppen sind Toluylendiisocyanat (TDI), Methylendiphenylisocyanat (MDI) und Naphthylendiisocyanat.

Der Begriff "Toluylendiisocyanat" bezeichnet Toluylen-2,4-diisocyanat (2,4-TDI), Toluylen-2,6-diisocyanat (2,6-TDI) sowie beliebige Gemische der beiden Isomere. Der Begriff "Methylendiphenylisocyanat" bezeichnet alle Isomeren des MDI, insbesondere 2,2'-Diphenylmethandiisocyanat, 2,4'-Diphenylmethandiisocyanat, 4,4'-Diphenylmethandiisocyanat, alle Gemische, die wenigstens zwei der vorgenannten Isomere enthalten, sowie mehrkernige Derivate des MDI.

Der Begriff "Naphthylendiisocyanat" bezeichnet, 1,4-Naphthylendiisocyanat, 1,5-Naphthylendiisocyanat und 1,6-Naphthylendiisocyanat sowie beliebige Gemische der vorgenannten Isomere.

Dem Fachmann ist bewusst, dass alle oben genannten Polyisocyanate auch als Oligomere vorliegen können, so dass die zu spaltende Urethanbindung zwischen einem oligomeren Polyisocyanat, das auf einem der oben genannten monomeren Diisocyanate basiert, und einem Alkohol ausgebildet ist.

Der Begriff "oligomeres Polyisocyanat" bezeichnet Polyisocyanat, die aus wenigstens zwei der oben genannten Diisocyanate aufgebaut sind. Die oligomeren Polyisocyanate können erfindungsgemäß insbesondere Uretdion-, Isocyanurat-, Allophanat-, Biuret-, Iminooxadiazindion- und/oder Oxadiazintrionstruktur aufweisen. Bevorzugt weisen die oligomeren Polyisocyanate mindestens eine der folgenden oligomeren Strukturtypen oder deren Gemische auf:

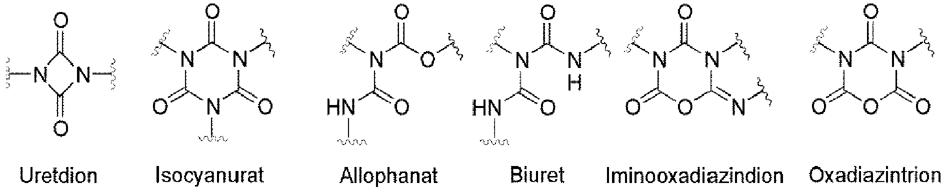

Bevorzugte Substrate der Urethanase der vorliegenden Erfindung sind Urethane, die aus den oben definierten aromatischen Polyisocyanaten erhalten werden können.

### Enzymatische Spaltung

Der Begriff "enzymatische Spaltung einer Urethangruppe" kennzeichnet, dass die oben beschriebene Spaltung einer Urethangruppe in Anwesenheit eines Polypeptids mit Urethanaseaktivität schneller abläuft als bei Inkubation mit dem Reaktionspuffer ohne Enzym unter denselben Reaktionsbedingungen oder bei Inkubation mit dem Reaktionspuffer unter denselben Bedingungen in Anwesenheit eines inaktiven Polypeptids. Als Modell für ein inaktives Polypeptid ist bovines Serumalbumin bevorzugt. Wenn in Anwesenheit eines zu testenden Polypeptids die Spaltung der Urethangruppe schneller abläuft als in einer ansonsten identischen Kontrolle mit BSA, besitzt besagtes Polypeptid Urethanaseaktivität wie in dieser Anmeldung verstanden.

### Nukleinsäure

In einer weiteren Ausführungsform betrifft die vorliegende Erfindung eine Nukleinsäure, die ein Polypeptid wie in SEQ ID NO.: 1 definiert oder eine Variante dieses Polypeptids kodiert. Bevorzugt steht die kodierende Nukleinsäuresequenz unter Kontrolle eines Promotors, der die Expression des Polypeptids in einem Pilz, einer Hefe oder einem Bakterium ermöglicht.

### Verwendung

In einer weiteren Ausführungsform betrifft die vorliegende Erfindung die Verwendung eines Polypeptids mit einer Aminosäuresequenz wie in SEQ ID NO.: 1 definiert und Varianten dieses Polypeptids, dadurch gekennzeichnet, dass das Polypeptid Urethanaseaktivität aufweist, zur enzymatischen Spaltung von Urethanbindungen.

Alle oben gegebenen Definitionen gelten - soweit nicht explizit anders definiert - auch für diese Ausführungsform. Die Verwendung besteht vorzugsweise darin, dass das erfindungsgemäße Enzym mit einer Verbindung, die wenigstens eine Urethanbindung enthält, in Kontakt gebracht wird. Dies geschieht vorzugweise unter Bedingungen, unter denen das Enzym seine Urethanaseaktivität zeigt.

Solche Bedingungen können den Ausführungsbeispielen dieser Anmeldung entnommen werden. Darüber hinaus ist der Fachmann ohne Probleme in der Lage, durch einfache Reihenversuche die Reaktionsbedingungen in einem System wie z.B. Temperatur, lonenstärke, Lösungsmittel und pH-Wert so anzupassen, dass das Enzym aktiv ist.

Für die erfindungsgemäße Urethanase liegt die Temperatur bei der Verwendung vorzugsweise im Bereich zwischen 10 °C und 70 °C. Hierbei wird ein höherer Temperaturbereich zwischen 30 °C und 70 °C bevorzugt, wenn die Reaktionsgeschwindigkeit wichtiger ist als die Stabilität des Enzyms. Umgekehrt wird ein Temperaturbereich zwischen 10 °C und 40 °C bevorzugt, wenn die Reaktionsgeschwindigkeit von geringerer Bedeutung ist.

Der pH liegt bei der Verwendung vorzugsweise zwischen 7 und 12, stärker bevorzugt zwischen 7 und 11 und am stärksten bevorzugt zwischen 8 und 11.

### Spaltung niedermolekularer Urethane

In noch einer weiteren Ausführungsform betrifft die vorliegende Erfindung ein Verfahren enthaltend den Verfahrensschritt der Behandlung eines niedermolekularen Urethans mit einem Polypeptid, welches Urethanaseaktivität aufweist und eine Aminosäuresequenz wie in SEQ ID Nr. 1 definiert hat oder mit einer Variante davon, wodurch das niedermolekulare Urethan gespalten wird.

Der Begriff "niedermolekular" bedeutet in diesem Zusammenhang, dass das Urethan höchstens vier, bevorzugt höchstens drei, stärker bevorzugt höchstens eine oder zwei Urethangruppen enthält.

Bevorzugt basieren die Strukturen der niedermolekularen Urethane formal auf der Ausbildung einer oder mehrerer Urethanbindungen zwischen einem Polyisocyanat und einem oder mehrerer niedermolekularer Alkohole.

Hierbei sind jene Polyisocyanate bevorzugt, die bereits weiter oben als Partner der durch die erfindungsgemäßen Urethanasen zu spaltenden Urethanbindungen genannt wurden. Aromatische Polyisocyanate, insbesondere Toluylen-2,4-diisocyanat (2,4-TDI), Toluylen-2,6-diisocyanat (2,6-TDI), 2,2'-Diphenylmethandiisocyanat (2,2'-MDI), 2,4'-Diphenylmethandiisocyanat (2,4'-MDI), 4,4'-Diphenylmethandiisocyanat (4,4'-MDI), die mehrkernigen Derivate der vorgenannten Diphenylmethandiisocyanate, 1,4-Naphthylendiisocyanat, 1,5-Naphthylendiisocyanat und 1,6-Naphthylendiisocyanat sind hierbei besonders bevorzugt.

Grundsätzlich ist als niedermolekularer Alkohol jede Verbindung mit wenigstens einer Hydroxylgruppe pro Molekül geeignet. Bevorzugt hat er ein Molekulargewicht von höchstens 700 g/Mol, stärker bevorzugt von höchstens 500 g/Mol und am stärksten bevorzugt von höchstens 200 g/Mol.

Weiterhin sind niedermolekulare Alkohole mit einer höheren Polarität bevorzugt. Besonders bevorzugt enthält der niedermolekulare Alkohol eine oder zwei Hydroxylgruppen pro Molekül. Der niedermolekulare Alkohol hat darüber hinaus bevorzugt einen Schmelzpunkt von höchstens 45 °C, stärker bevorzugt von höchstens 20 °C.

Stärker bevorzugt sind folglich Alkohole mit zwei Hydroxylgruppen, einem Molekulargewicht von höchstens 500 g/Mol und einem Schmelzpunkt von höchstens 45 °C.

In einer besonders bevorzugten Ausführungsform der vorliegenden Erfindung ist der niedermolekulare Alkohol ausgewählt aus der Gruppe bestehend aus Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol und Polyethylenglykol 400. Stärker bevorzugt ist er ausgewählt aus der Gruppe bestehend aus Ethylenglykol, Diethylenglykol, Propylenglykol, Dipropylenglykol, Methylglykol und Triethylenglykol. Ganz besonders bevorzugt ist der niedermolekulare Alkohol Diethylenglykol.

Ein für das erfindungsgemäße Verfahren besonders bevorzugtes niedermolekulares Urethan ist durch Ausbildung einer Urethanbindung zwischen einem der im vorangehenden Abschnitt genannten niedermolekularen Alkohole, insbesondere Diethylenglykol, und erhältlich.

Die niedermolekularen Urethane können auf unterschiedliche Weisen erhalten werden. In einer bevorzugten Ausführungsform der vorliegenden Erfindung werden die Esterbindungen eines Polyester-Polyurethans gespalten, so dass Carbonsäuren und niedermolekulare Urethane freigesetzt werden.

In einer bevorzugten Ausführungsform der Erfindung erfolgt die Spaltung der Estergruppen erfolgt vorzugsweise enzymatisch. Hierbei werden bevorzugt Lipasen (EC 3.1.1.3) oder Cutinasen (EC 3.1.1.74) eingesetzt.

In einer weiteren bevorzugten Ausführungsform der vorliegenden Erfindung handelt es sich bevorzugt um solche niedermolekularen Urethane, die durch die chemische Spaltung eines Polyether- oder Polyester-Polyurethans erhalten werden. Bei dieser chemischen Spaltung handelt es sich vorzugweise um eine sogenannte "Alkoholyse" oder "Glykolyse", bei der das Polyether- oder Polyester-Polyurethan mit einem der oben genannten niedermolekularen Alkohole zur Reaktion gebracht, so dass die Etherkomponente des Polyether-Polyurethans bzw. das Polyesterpolyol eines Polyester-Polyurethans durch den niedermolekularen Alkohol ersetzt werden.

Eine besonders bevorzugte Ausführungsform zur Bereitstellung von niedermolekularen Urethanen aus Polyether-Polyurethanen ist in WO 2021/032513 beschrieben. In diesem Verfahren werden Polyetherpolyurethane mit einem niedermolekularen Alkohol so umgesetzt, dass die Polyetherkomponente des Polyetherpolyurethans freigesetzt wird und eine Urethanbindung zwischen dem verwendeten niedermolekularen Alkohol und den zum Aufbau des Polyether-Polyurethans verwendeten Isocyanaten ausgebildet wird. Somit handelt es sich bei den erfindungsgemäß zu spaltenden niedermolekularen Urethanen um Verbindungen, die formal durch Bildung einer Urethanbindung zwischen einem Polyisocyanat, bevorzugt einem der weiter oben in dieser Anmeldung aufgeführten aliphatischen, aromatischen, araliphatischen oder cycloaliphatischen Polyisocyanate, und einem niedermolekularen Alkohol erhalten werden.

### Polyester-Polyurethan

Der Begriff "Polyester-Polyurethan" bezeichnet ein Polyurethan, dessen Polyolkomponente Polyesterpolyole enthält. Bevorzugt sind wenigstens 40 Gew.-% der in der Polyolkomponente enthaltenen Hydroxylgruppen Bestandteile von Polyesterpolyolen. Stärker bevorzugt sind dies wenigstens 60 Gew.-%, noch stärker bevorzugt wenigstens 80 Gew.-% und am stärksten bevorzugt wenigstens 95 gew.-%. Das Polyester-Polyurethan kann geschäumt oder nicht geschäumt vorliegen.

Das Polyester-Polyurethan enthält als Isocyanatkomponente wenigstens ein aromatisches, aliphatisches oder cycloaliphatisches Isocyanat. Bevorzugt enthält das Polyurethan aromatische Isocyanate. Das wenigstens eine aromatische Polyisocyanat ist besonders bevorzugt aus der weiter oben gegebenen Liste ausgewählt.

Der Begriff "Polyesterpolyol" ist dem Fachmann bekannt, es handelt sich um Polyester, die im Durchschnitt pro Molekül wenigstens 1,5, bevorzugt wenigstens 1,8 und stärker bevorzugt wenigstens 2,0 Hydroxylgruppen enthalten. Besonders bevorzugt weisen die in dem abzubauenden Polyurethan enthaltenen Polyesterpolyole Funktionalität zwischen 1,5 und 6,0 auf. Sie enthalten als Aufbaukomponenten aromatische und/oder aliphatische Polyole sowie aromatische und/oder aliphatischen Polycarbonsäuren in beliebiger Kombination. Ebenfalls bevorzugt sind Polyesterpolyole, die durch Ringöffnungspolymerisation eines Lactons erhalten werden.

### Polyether-Polyurethan

Der Begriff "Polyether-Polyurethan" bezeichnet ein Polyurethan, dessen Polyolkomponente Polyetherpolyole enthält. Bevorzugt sind wenigstens 40 Gew.-% der in der Polyolkomponente enthaltenen Hydroxylgruppen Bestandteile von Polyetherpolyolen. Stärker bevorzugt sind dies wenigstens 60 Gew.-%, noch stärker bevorzugt wenigstens 80 Gew.-% und am stärksten bevorzugt wenigstens 95 Gew.-%. Es ist erfindungsgemäß möglich, dass ein Polyetherpolyurethan unter Einhaltung der vorgenannten Mengenanteile der Polyetherpolyole auch noch weitere Polyole als Aufbaukomponenten enthält. Dies sind vorzugsweise Polyesterpolyole.

Der Begriff "Polyetherpolyol" ist dem Fachmann gut bekannt. Hierbei handelt es sich um Polyether mit einer durchschnittlichen Hydroxylfunktionalität zwischen 1,5 und 6,0. Bevorzugt ist das im Polyetherpolyurethan enthaltene Polyetherpolyol ein Polyadditionsprodukt eines oder mehrerer Alkylenoxide mit 2 bis 4 Kohlenstoffatomen unter Verwendung mindestens eines Startermoleküls, das 2 bis 8, vorzugsweise 2 bis 6, reaktive Wasserstoffatome gebunden enthält.

Bevorzugte Alkylenoxide sind Styroloxid, Ethylenoxid, Propylenoxid, Tetrahydrofuran, Butylenoxid, Epichlorhydrin. Stärker bevorzugt sind 1,3-Propylenoxid, 1,2- bzw. 2,3-Butylenoxid und Styroloxid. Besonders bevorzugt sind Ethylenoxid und 1,2-Propylenoxid. Die Alkylenoxide können einzeln, alternierend nacheinander oder als Mischungen verwendet werden.

Die "Behandlung" des niedermolekularen Urethans erfolgt unter Bedingungen, unter denen das eingesetzte Enzym Urethanaseaktivität zeigt. Unter diesen Bedingungen entstehen als Reaktionsprodukte der enzymatischen Spaltung des niedermolekularen Urethans ein niedermolekularer Alkohol bzw. ein Gemisch niedermolekularer Alkohole und ein Amin oder ein Gemisch von Aminen. Die chemische Struktur der entstehenden Amine ist von der Art des zur Synthese des Urethans eingesetzten Polyisocyanats abhängig. Es werden Amine freigesetzt, die von den eingesetzten Polyisocyanaten durch Anlagerung von Wasser und nachfolgende Abspaltung von CO₂ abgeleitet werden können.

In noch einer weiteren Ausführungsform betrifft die vorliegende Erfindung die Verwendung wenigstens eines der erfindungsgemäßen Polypeptide zur Spaltung einer Urethanbindung in niedermolekularen Urethanen.

### Abbau von Polyester-Urethanen in niedermolekulare Abbauprodukte

In noch einer weiteren Ausführungsform betrifft die vorliegende Erfindung ein Verfahren zum Abbau von Polyester-Polyurethanen in niedermolekulare Abbauprodukte enthaltend die Schritte
a) Spaltung der im Polyester-Polyurethan enthaltenen Estergruppen; und
b) Behandlung des Polyurethans mit einem Polypeptid, welches Urethanaseaktivität aufweist und eine Aminosäuresequenz wie in SEQ ID NO.: 1 definiert hat oder einer Variante davon;
mit der Maßgabe, dass die Verfahrensschritte a) und b) in beliebiger Reihenfolge oder auch gleichzeitig durchgeführt werden können.

Vorzugsweise wird der Verfahrensschritt a) vor dem Verfahrensschritt b) durchgeführt.

Soweit nicht anders angegeben gelten alle weiter oben in dieser Anmeldung gegebenen Definition auch für diese Ausführungsform. Dies gilt insbesondere für die Behandlung des Polyurethans mit einer Urethanase in Verfahrensschritt b).

In einer bevorzugten Ausführungsform der Erfindung wird Verfahrensschritt a) mit einer Lipase oder Cutinase durchgeführt. Stärker bevorzugt wird eine Lipase verwendet, die in der Lage ist, Tributyrin zu spalten. Verfahrensschritt a) wird vorzugsweise unter Reaktionsbedingungen durchgeführt, bei denen die verwendete Lipase oder Cutinase Aktivität zeigt. Solche Bedingungen können durch Routineexperimente mit gängigen biochemischen Methoden bestimmt werden.

In einer anderen, ebenfalls bevorzugten Ausführungsform erfolgt der Verfahrensschritt a) chemisch.

Das Polyurethan enthält als Isocyanatkomponente wenigstens ein aromatisches, aliphatisches oder cycloaliphatisches Isocyanat. Bevorzugt enthält das Polyurethan aromatische Isocyanate.

Die niedermolekularen Abbauprodukte der polyesterbasierten Polyurethane weisen vorzugsweise ein Molekulargewicht von höchstens 1.000 g/mol auf. Es handelt sich vorzugsweise um
(i) Amine, die von den bei der Herstellung des betreffenden Polyurethans eingesetzten Isocyanaten abgeleitet sind, Beispielsweise im Falle von 2,4- Toluylendiisocyanat um das 2,4-Toluylendiamin; und
(ii) Alkohole und Carbonsäuren, die zum Aufbau der für die Synthese des betreffenden Polyurethans eingesetzten Polyesterpolyole eingesetzt wurden.

Unter einem "Polyol", das in dem in diesem Abschnitt definierten Verfahren als niedermolekulares Abbauprodukt entsteht, wird in dieser Anmeldung jede Verbindung mit wenigstens zwei Hydroxylgruppen verstanden. Besagtes Polyol hat vorzugsweise ein Molekulargewicht von höchstens 300 g/mol. Bevorzugte Polyole, die niedermolekulare Abbauprodukte der polyesterbasierten Polyurethane sind, sind ausgewählt aus der Gruppe bestehend aus Ethylenglykol, Diethylenglykol, Triethylenglykol, Propylenglykol, 1,3-Pentandiol, 1,4-Butandiol, 1,6,-Hexandiol, 1,2-Di-Propylenglykol, Neopentylglykol, Glycerin, 1,1,1-Trimethylolpropan, Saccharose, Sorbitol und Pentaerythritol.

Unter einer "Polycarbonsäure", die in dem in diesem Abschnitt definierten Verfahren als niedermolekulares Abbauprodukt entsteht, wird in dieser Anmeldung jede Verbindung verstanden, die wenigstens zwei Carboxylgruppen enthält. Besagte Polycarbonsäure hat vorzugsweise ein Molekulargewicht von höchstens 300 g/mol. Bevorzugte Polycarbonsäuren, die niedermolekulare Abbauprodukte der polyesterbasierten Polyurethanschäume sind, sind ausgewählt aus der Gruppe bestehend aus Bernsteinsäure, Glutarsäure, Adipinsäure, Itakonsäure, Phthalsäure, Isophthalsäure, Terephthalsäure und Benzoltricarbonsäure.

Unter einem "Polyamin", das in dem in diesem Abschnitt definierten Verfahren als niedermolekulares Abbauprodukt entsteht, wird in dieser Anmeldung jede Verbindung verstanden, die wenigstens zwei Aminogruppen enthält. Besagtes Polyamin hat vorzugsweise ein Molekulargewicht von höchstens 300 g/mol. Bevorzugte Polyamine, die niedermolekulare Abbauprodukte der polyesterbasierten Polyurethanschäume sind, sind ausgewählt aus der Gruppe bestehend aus 4,4'-Diaminodiphenylmethan, 2,4'-Diaminodiphenylmethan, 2,2'-Diaminodiphenylmethan, den mehrkernigen Derivate der vorgenannten Diaminodiphenylymethanverbindungen, 2,4-Toluylendiamin, 2,6-Toluylendiamin, 1,4-Naphthylendiamin, 1,5-Naphthylendiamin, 1,6-Naphthylendiamin, Hexamethylendiamin, Isophorondiamin, Xylylendiamin, Pentamethylendiamin, para-Phenylendiamin, Butyldiamin und H12-Methylendiamin. Besonders bevorzugt sind die Polyamine ausgewählt aus der Gruppe bestehend aus 4,4'-Methylendiamin, 2,4'-Methylendiamin, 2,2'-Methylendiamin, 1,5-Naphthylendiamin, 2,4-Toluylendiamin und 2,6-Toluylendiamin.

### Abbau von Polyether-Polyurethan

In noch einer weiteren Ausführungsform betrifft die vorliegende Erfindung ein Verfahren enthaltend die Schritte
a) Umurethanisierung eines Polyetherpolyurethans mit wenigstens einem niedermolekularen Alkohol, wobei Polyetherpolyole und niedermolekulare Urethane entstehen; und
b) die enzymatische Spaltung der in Verfahrensschritt a) entstandenen niedermolekularen Urethane mit einem Polypeptid, welches Urethanaseaktivität aufweist und eine Aminosäuresequenz hat wie in SEQ ID NO.: 1 definiert oder einer Variante davon.

Der Verfahrensschritt a) hat zwei Ziele: (i) Das zur Synthese des Polyurethans verwendete Polyetherpolyol soll als isolierbare Verbindung aus dem Polyurethan freigesetzt werden. (ii) Das zur Synthese des Polyurethans verwendete Isocyanat soll als Bestandteil eines niedermolekularen Urethans vorliegen. Das besagte niedermolekulare Urethan ist - im Gegensatz zum Polyurethan mit seinem hohen Molekulargewicht - durch sein geringeres Molekulargewicht und die dadurch bessere Löslichkeit als Substrat für die in Verfahrensschritt b) erfolgende enzymatische Spaltung gut geeignet. Soweit nicht anders angegeben gelten alle weiter oben in dieser Anmeldung gegebenen Definition auch für diese Ausführungsform. Dies gilt insbesondere für die enzymatische Spaltung der niedermolekularen Urethane in Verfahrensschritt b).

In Verfahrensschritt b) werden durch enzymatische Spaltung der niedermolekularen Urethane ein Amin und der in Verfahrensschritt a) zur Umurethanisierung eingesetzte niedermolekulare Alkohol freigesetzt. Zusätzlich wird in diesem Verfahrensschritt CO₂ freigesetzt. Diese Verbindungen können durch geeignete Trennungsverfahren isoliert und anschließend weiter verwendet werden. Hierbei ist es bevorzugt, den freigesetzten niedermolekularen Alkohol erneut für die in Verfahrensschritt a) erfolgende Umurethanisierung einzusetzen. Das freigesetzte Amin steht als reiner und gut definierter Ausgangsstoff für neue Synthesen zur Verfügung.

Die grundsätzlich geeigneten Reaktionsbedingungen und Katalysatoren für die Umurethanisierung in Verfahrensschritt a) sind in Simon et al. (2018), Waste Management, 76: 147-171 beschrieben. Verfahrensschritt a) wird bei Temperaturen zwischen 140 °C und 300 °C, bevorzugt zwischen 160 °C und 270 °C durchgeführt. Das Gewichtsverhältnis des niedermolekularen Alkohols zum Polyetherurethan liegt zwischen 2 : 1 und 1 : 17. Als Katalysator eignen sich insbesondere AlkalimetallHydroxide, Erdalkalimetallhydroxide, Alkalimetallsalze von Carbonsäuren (insbesondere Acetate), Erdalkalimetallsalze von Carbonsäure (insbesondere Acetate), Lewis-Säuren (wie insbesondere Dibutylzinndilaurat), organischen Amine (wie insbesondere Diethanolamin), organometallische Verbindungen (wie insbesondere Titantetrabutanolat) und Zinnverbindungen (wie insbesondere Zinnoctoat). Die Umurethanisierung wird bevorzugt bei Temperaturen im Bereich von 160 °C bis 270 °C in Gegenwart von 0,1 Massen-% bis 5 Massen-% Katalysator, bezogen auf die Masse des zugegebenen Polyurethanprodukts, durchgeführt.

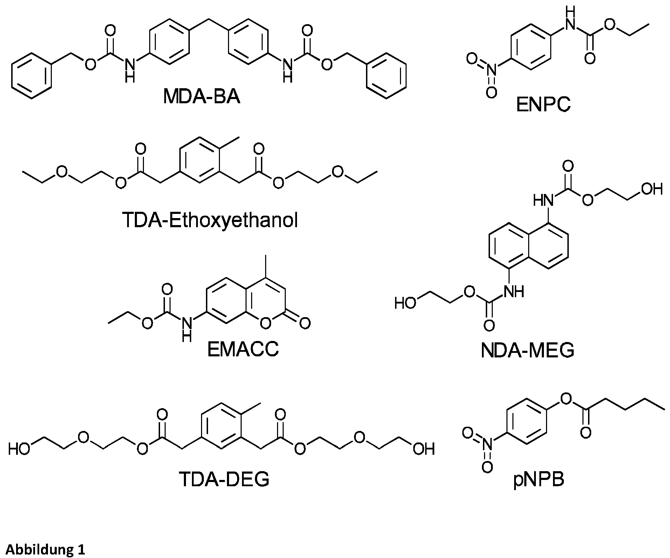

- **Abbildung 1 zeigt:**: Genutzte Enzymsubstrate. pNPB: 4-Nitrophenylbutyrate; ENPC: Ethyl-4-nitrophenylcarbamate; MDA-BA: Carbamat von 4,4'-MDI reagiert mit Benzylalkohol; TDA-Ethoxyethanol: Carbamat von Toluoldiisocyanat (TDI) reagiert mit Ethoxyethanol; TDA-DEG: Carbamat von TDI reagiert mit Diethylenglykol; NDA-MEG: Carbamat von NDI reagiert mit Ethylenglykol; EMACC: Carbamat aus der Reaktion von 7-Amino-4-methylcoumarin mit Ethylchlorformiat.

Die folgenden Ausführungsbeispiele dienen nur dazu, die Erfindung zu erläutern. Sie sollen den Schutzumfang der Patentansprüche in keiner Weise beschränken.

### Beispiele

### Beispiel 1: Aktivitätsbestimmung mit EMACC

### Enzymherstellung

Urethanasegene wurden in den pET-26-Expressionsvektor direkt hinter die Ndel-Schnittstelle kloniert. *E. coli* BL21(DE3) wurden zur Expression mit den entsprechenden Plasmiden transformiert. Eine Einzelkolonie wurde zur Inokulation der Übernachtkultur in LB-Medium mit 1% (w/v) Glukose und 50 µg/mL Kanamycin verwendet. Für die Hauptkultur wurden 200 ml ZYP-5052 mit 50 µg/mL Kanamycin mit 1 mL der Übernachtkultur angeimpft und für 4 h bei 37 °C bei 100 rpm in Schikanekolben inkubiert. Anschließend wurde die Temperatur auf 20 °C reduziert, bevor die Kulturen durch Zentrifugation für 20 min bei 4500 g und 4 °C geerntet wurden. Zellpellets wurden bis zur Reinigung der Proteine bei -20 °C gelagert.

Zur Reinigung wurden 15 mL Lysepuffer (50 mM Natriumphosphat (NaPi), pH 8,0, 300 mM NaCl, 10 mM Imidazol) zum Zellpellet gegeben. Der Aufschluss erfolgte per Ultraschall auf Eis über zwei Zyklen (3 min, 50% pulse, 50% Power). Zur Klärung des Lysates wurde bei 10.000 g und 4 °C für 40 - 60 min zentrifugiert. Die Überstände wurden bis zum Beladen der IMAC-Säule auf Eis gelagert.

IMAC-Harz (1 ml, ROTI^{®}Garose His Beads, Nickel-Form) wurde mit Lysepuffer equilibriert. Anschließend wurde das Lysat auf die Säulen aufgetragen gefolgt von einem Waschschritt mit mindestens zehn Säulenvolumen Waschpuffer (50 mM NaPi, pH 8,0, 300 mM NaCl, 20 mM Imidazol). Anschließend wurden die Proteine mit 15 mL Elutionspuffer (50 mM NaPi, pH 8,0, 300 mM NaCl, 250 mM Imidazol) eluiert. Das Volumen der Elutionsfraktion wurde über Vivaspin-20-Ultrafiltrationseinheiten (10 kDa MWCO) auf 2,5 mL reduziert. Anschließend wurden die Proteine unter Verwendung von PD10-Säulen in Lagerpuffer (10 mM NaPi, pH 8,0, 100 mM NaCl) umgepuffert.

### EMACC Assay

Zur Bestimmung der Urethanaseaktivität wurde ein fluorogenes Substrat aus 7-Amino-4-methylcoumarin (AMC) und Ethylchlorformiat synthetisiert. Das so gebildete Ethylcarbamat von AMC (EMACC) lag als feines Pulver vor. Die Bestimmung der spezifischen Aktivität erfolgte unter Standardbedingungen (50 mM NaPi, pH 8,0, 30 °C, 100 µM EMACC). EMACC wurde aus einem 50 mM Stock in DMSO frisch zugegeben. Gereinigte Urethanasen wurden vor der Zugabe zum Assay in Lagerpuffer verdünnt, sodass sich ein linearer Anstieg der Absorption bei 365 nm über 10 - 30 min ergab. Die Messung erfolge in 96-well-Platten in einem auf 30 °C vorgeheizten Plattenphotometer und die spezifische Aktivität wurde aus dem linearen Anstieg der Absorption in den ersten Minuten berechnet. Die Ergebnisse sind in

Tabelle 1 dargestellt.

**Tabelle 1: Spezifische Aktivität der Urethanase gegenüber EMACC**

| **Urethanase** | **Spezifische Aktivität gegen EMACC [µmol/min/mg Enzym]** |
|---|---|
| UMG-SP-8 (SEQ ID NO.: 1) | 0,749 ± 0,012 |

### Beispiel 2: Screening gegenüber weiteren Substraten

### Enzymherstellung

Für die Herstellung der Enzympräparate wurden jeweils 4,5 mL LB-Medium mit 1 % (w/v) Glucose mit Einzelkolonien von *E. coli* BL21(DE3) pET26b_UMG-8 inokuliert und über Nacht bei 37 °C und 200 rpm im Inkubationsschüttler inkubiert. Sämtliche Kulturen wurden mit 50 µg/mL Kanamycin versetzt. Anschließend wurden jeweils 200 mL ZYP-5052 Medium mit 200 µL der Übernachtkulturen inokuliert. Die Kultivierung erfolgte für 4 h bei 37°C und 200 rpm gefolgt von 24 h bei 20°C und 200 rpm im Inkubationsschüttler. Die Zellen wurden durch Zentrifugation bei 20.425 g und 4 °C für 20 min in der Großraumzentrifuge abgetrennt. Die Zellpellets wurden in jeweils 6 mL Aufschlusspuffer (20 mM Ammoniumacetat, 0,4 % n-Dodedecyl-β-Maltosid, 1 % Lysozym, 1 µL/mL Benzonase (Sigma-Aldrich Chemie GmbH, Taufkirchen)) suspendiert und für 30 min bei Raumtemperatur auf dem Orbitalschüttler inkubiert. Anschließend wurde die Ganzzellextraktlösung bei -80 °C eingefroren und gefriergetrocknet. Das Lyophilisat wurde bei 4 °C gelagert.

### Enzymreaktionen

Die Substrate ENPC, MDA-BA, NDA-MEG, TDA-Ethoxyethanol und TDA-DEG wurden aus den entsprechenden Isocyanaten und Alkoholen synthetisiert indem die Isocyanate mit einem Überschuss an Alkohol zur Reaktion gebracht wurden. Zur Bestätigung der Aktivität der Urethanasen wurden EMACC und 4-Nitropenylbutyrat (pNPB) genutzt. Die Enzymsubstrate sind in **Fehler! Verweisquelle konnte nicht gefunden werden.** dargestellt.

Je eine Spatelspitze (~3 mg) der lyophilisierten Ganzzellextrakte wurde in 800 µL 100 mM KPi mit 100 mM NaCl pH 7,5 suspendiert. Die Enzymlösung wurde 1:10 in das Reaktionsgemisch verdünnt, welches den gleichen Puffer enthielt. Es wurden Substrat-Stocks der Carbamate mit einer Konzentration von 5 - 340 mg/mL in DMSO hergestellt. Diese wurden 1:10 in das Reaktionsgemisch verdünnt, sodass die DMSO-Endkonzentration bei 10 % (v/v) lag. Die Ansätze wurden mit einem Gesamtvolumen von 500 µL hergestellt. Die Inkubation erfolgte für einen Tag bei 30°C und 800 - 1000 rpm gefolgt von einen Tag bei 40°C und 800-1000 rpm im Thermoschüttler. Nach der Inkubation wurde zur Verbesserung der Löslichkeit von Substraten und Produkten 500 µL Acetonitril zugesetzt. Die Proben wurden durch Zentrifugation durch eine 0,2 µm PVDF-Filterplatte filtriert und für die HPLC-Analytik eingesetzt. Für die Substrate EMACC und pNPB lag das Gesamtvolumen der Reaktion bei 200 µL und die Fluoreszenz (EMACC, Anregung 365 nm, Emission 440 nm) bzw. Absorption (pNPB, 410 nm) wurde zu verschiedenen Zeitpunkten im Plattenphotometer Varioskan Lux (Thermo Fisher Scientific Inc., Waltham, Massachusetts, USA) gemessen.

### HPLC-Analytik

Die Hochdruckflüssigchromatographie wurde an einem Gerät der 1260 Infinity II Serie von Agilent Technologies (Santa Clara, USA) mit *Multisampler* und DAD (*diode array detector*) für UV und den sichtbaren Bereich des Lichtes durchgeführt. Für alle Messungen wurde die Säule "Zorbax Eclipse Plus-C18" mit einer Partikelgröße von 5 µm und den Abmessungen 4,6 x 150 mm (Agilent Technologies, Santa Clara, USA) mit entsprechender Vorsäule verwendet. Bei allen Methoden wurden 5 µL Probe injiziert und die Säule auf 40 °C temperiert. Der Fluss lag generell bei 1,0 mL/min.

Zur Detektion von aromatischen Aminen wurde die Methode "ER_Juni2021-2" verwendet. Aromatische Amine konnten wegen der hohen Eigenabsorption ohne Derivatisierung mit dieser Methode bei 210 nm und 232 nm quantifiziert werden. Als Laufmittel A wurde ddH₂O mit 5 % (v/v) Acetonitril und als Laufmittel B wurde Acetonitril mit 5 % (v/v) ddH₂O verwendet. Die Analyse der Daten erfolgte mit der Software "OpenLAB CDS 2.4" in der Version 2.204.0.661 (Agilent Technologies, Santa Clara, USA).

**Tabelle 2: HPLC-Gradient**

| t [min] | Laufmittel A [%] | Laufmittel B [%] |
|---|---|---|
| 0,00 | 100,00 | 0,00 |
| 2,00 | 100,00 | 0,00 |
| 10,00 | 0,00 | 100,00 |
| 11,00 | 0,00 | 100,00 |
| 11,50 | 100,00 | 0,00 |
| 15,00 | 100,00 | 0,00 |

### Ergebnisse

Die Klassifizierung der hydrolytischen Aktivität der Urethanasen gegenüber den getesteten Carbamaten erfolgte anhand der Peakfläche der gebildeten Produkte im Chromatogramm in die Kategorien keine Aktivität (-), wenig Aktivität (+), mittlere Aktivität (++) und hohe Aktivität (+++). Es zeigte sich, dass die Urethanase UMG-8 ein breites Spektrum an Substraten akzeptiert. Die getesteten Substrate sind in **Fehler! Verweisquelle konnte nicht gefunden werden.** dargestellt.

**Tabelle 3: Aktivität der Urethanase gegenüber verschiedenen Carbamaten. Aktivität ist in die Kategorien "keine Aktivität" (-), "wenig Aktivität" (+), "mittlere Aktivität" (++) und "hohe Aktivität" (+++) anhand der HPLC-Peakfläche eingeteilt.**

| **Substrat** | **UMG-8** |
|---|---|
| EMACC | +++ |
| pNPB | +++ |
| ENPC | ++ |
| MDA-BA | ++ |
| NDA-MEG | ++/+++ |
| TDA-Ethoxyethanol | +++ |
| TDA-DEG | ++ |

### Beispiel 3: Optimierung der Reaktionsbedingungen

Zur Bestimmung der pH-Optima wurde Enzymaktivität analog zu Beispiel 1 bestimmt, mit der Anpassung, dass die Reaktion in verschiedenen Puffern (100 mM) durchgeführt wurde. Es wurden Puffer mit pH 4,0, 5,0 und 6,0 (Citrat), 7,0, 8,0 und 9,0 (Bis-Tris-Propan) und 10,0 (CHES) verwendet. Für die pH-Werte 10, 11, 12 und 13 wurde ein Puffer aus 100 mM Natriumphosphat und 100 mM Natriumcarbonat und abweichend von Beispiel 1 mit 200 mM EMACC und 10% (v/v) DMSO verwendet. Temperaturoptima wurden bestimmt, indem die Freisetzung von AMC aus 100 µM EMACC in 50 mM NaPi (pH 8,0) bei 30, 35, 40, 45, 50, 55, 60, 65 und 70 °C gemessen wurde. Die Lösemittelstabilität wurde bestimmt, indem die Hydrolyse von EMAC unter den in Beispiel 1 angegebenen Bedingungen bei 0, 10, 20, 30, 40 und 50 % (v/v) DMSO quantifiziert wurde. Die hier bestimmten Aktivitätsoptima für pH, Temperatur und DMSO-Konzentration sind in

| **Urethanase** | **pH-Optimum** | **T-Optimum (°C)** | **Optimale DMSO-Konzentration (%v/v)** |
|---|---|---|---|
| **UMG-SP-8** | 10 | 70 | 0 |

Tabelle 4 zusammengefasst. Die Abhängigkeit der Aktivität ist in

**Tabelle 4: Optimale Reaktionsbedingungen für Urethanase.**

| **Urethanase** | **pH-Optimum** | **T-Optimum (°C)** | **Optimale DMSO-Konzentration (%v/v)** |
|---|---|---|---|
| **UMG-SP-8** | 10 | 70 | 0 |

**Tabelle 5: Abhängigkeit der Aktivität der Urethanase vom pH-Wert. In beiden Datensets war die Aktivität bei pH 10 am höchsten, sodass auf diesen Wert normiert wurde. Die Aktivitätsdaten von pH 12 und pH 13 wurden nicht dargestellt, da die Fluoreszenz von AMC nur im Bereich zwischen pH 3 und pH 12 konstant ist.**

| | **pH** | | | | | | | |
|---|---|---|---|---|---|---|---|---|
| | **4** | **5** | **6** | **7** | **8** | **9** | **10** | **11** |
| **UMG-SP-8** | 0% | 0% | 0% | 41% | 77% | 82% | 100% | 86% |

### Temperaturstabilität

Zur Ermittlung der Langzeit-Temperaturstabilität wurden Lösungen der gereinigten UMG-8 mit einer Proteinkonzentration von 0,5 mg/mL in 100 mM KPi pH 7,5 hergestellt. Jeweils 100 µL wurden im Thermocycler Biometra TAdvanced Basis (Analytik Jena GmbH, Jena) bei 20°C, 25,7 °C, 29,4 °C, 36,9 °C, 40,6 °C, 44,3 °C und 50 °C für 12 h und anschließend bis zur Aktivitätsmessung bei 4°C inkubiert. Die Deckelheizung wurde auf 99°C temperiert. Eine Referenzprobe wurde bei 4 °C inkubiert. Die Restaktivität der Enzyme wurde mittels pNPB-Assay bestimmt. Dazu wurden 21 µL pNPB mit 4,979 mL DMSO vermischt. Diese Lösung wurde 1:10 in 100 mM KPi pH 7,5 verdünnt. 20 µL Enzymlösung (UMG-8 1:5 in 100 mM KPi pH 7,5 verdünnt) wurden in einer Mikrotiterplatte vorgelegt und die Reaktion wurde durch Zugabe von 180 µL pNPB in 100 mM KPi pH 7,5 mit 10 % DMSO gestartet. Bei der Hydrolyse des pNPBs durch die Urethanasen wird para-Nitrophenol frei, welches durch die Messung der Absorption bei 410 nm im Plate Reader Varioskan Lux (Thermo Fisher Scientific Inc., Waltham, Massachusetts, USA) quantifiziert wurde. Die Ergebnisse sind in Tabelle 6 dargestellt.

**Tabelle 6: Spezifische Aktivität der Urethanase gegenüber pNPB in U/mg nach Inkubation für 12 h bei 20°C, 25,7 °C, 29,4 °C, 36,9 °C, 40,6 °C, 44,3 °C und 50 °C und bei 4 °C als Referenz.**

| **Inkubationstemperatur** | **4 °C** | **20 °C** | **25,7 °C** | **29,4 °C** | **36,9 °C** | **40,6 °C** | **44,3 °C** | **50 °C** |
|---|---|---|---|---|---|---|---|---|
| **Enzym** | **Spezifische Aktivität [µmol/min/mg Enzym]** | | | | | | | |
| UMG-8 | 6,73 | 6,50 | 7,12 | 6,42 | 3,23 | 0 | 0 | 0 |

## Patentansprüche

1. Polypeptid mit einer Aminosäuresequenz wie in SEQ ID NO.: 1 definiert oder eine Variante davon, **dadurch gekennzeichnet, dass** das Polypeptid Urethanaseaktivität aufweist.

2. Polypeptid gemäß Anspruch 1, wobei die Variante durch die Hinzufügung, Deletion oder Austausch von bis zu 15 % der im jeweiligen Polypeptid enthaltenen Aminosäuren erhalten werden.

3. Verwendung eines Polypeptids mit einer Aminosäuresequenz wie in SEQ ID NO.: 1 oder einer Variante dieses Polypeptids, **dadurch gekennzeichnet, dass** das Polypeptid Urethanaseaktivität aufweist, zur enzymatischen Spaltung von Urethanbindungen.

4. Verwendung gemäß Anspruch 3, wobei die Urethangruppe aromatisch gebunden ist.

5. Nukleinsäure, die ein Polypeptid wie in SEQ ID NO. 1 definiert oder eine Variante des vorgenannten Polypeptids kodiert.

6. Verfahren enthaltend den Verfahrensschritt der Behandlung eines niedermolekularen Urethans mit einem Polypeptid, welches Urethanaseaktivität aufweist und eine Aminosäuresequenz wie in SEQ ID Nr. 1 definiert hat, oder mit einer Variante davon, wodurch das niedermolekulare Urethan gespalten wird.

7. Das Verfahren nach Anspruch 6, wobei wenigstens ein Polyamin ausgewählt aus der Gruppe bestehend aus 4,4'-Diaminodiphenylmethan, 2,4'-Diaminodiphenylmethan, 2,2'-Diaminodiphenylmethan, den mehrkernigen Derivate der vorgenannten Diaminodiphenylymethanverbindungen, 2,4-Toluylendiamin, 2,6-Toluylendiamin, 1,4-Naphthylendiamin, 1,5-Naphthylendiamin und 2,6-Toluylendiamin entsteht.

8. Das Verfahren nach Anspruch 6 oder 7, wobei ein Alkohol mit wenigstens zwei Hydroxylgruppen und einem Molekulargewicht von höchstens 700 g/Mol freigesetzt wird.

9. Das Verfahren nach Anspruch 8, wobei wenigstens ein Alkohol ausgewählt aus der Gruppe bestehend aus Ethylenglykol, Diethylenglykol, Propylenglykol, Dipropylenglykol, Methylglykol, Triethylenglykol, Glycerin, 2-Methyl-1,3-Propandiol, 1,4-Butandiol, 1,5-Pentandiol, 1,6-Hexandiol und Polyethylenglykol 400 freigesetzt wird.

10. Verwendung wenigstens eines Polypeptids, welches Urethanaseaktivität aufweist und eine Aminosäuresequenz wie in SEQ ID NO.: 1 definiert hat oder eine Aminosäuresequenz mit wenigstens 85 % Sequenzidentität zu der vorgenannten Sequenz hat, zur Spaltung einer Urethanbindung in niedermolekularen Urethanen.

11. Verfahren zum Abbau von Polyester-Polyurethanen in niedermolekulare Abbauprodukte enthaltend die Schritte
a) Spaltung der im Polyester-Polyurethan enthaltenen Estergruppen; und
b) Behandlung des Polyurethans mit einem Polypeptid, welches Urethanaseaktivität aufweist und eine Aminosäuresequenz wie in SEQ ID NO.: 1 definiert hat oder einer Variante davon;
mit der Maßgabe, dass die Verfahrensschritte a) und b) in beliebiger Reihenfolge oder auch gleichzeitig durchgeführt werden können.

12. Verfahren enthaltend die Schritte
a) Umurethanisierung eines Polyetherpolyurethans mit wenigstens einem niedermolekularen Alkohol, wobei Polyetherpolyole und niedermolekulare Urethane entstehen; und
b) die enzymatische Spaltung der in Verfahrensschritt a) entstandenen niedermolekularen Urethane mit einem Polypeptid, welches Urethanaseaktivität aufweist und eine Aminosäuresequenz hat wie in SEQ ID NO.: 1 definiert oder einer Variante davon.
